(19) Europäisches Patentamt
European Patent Office
Office européen des brevets

(11) Veröffentlichungsnummer: **0 295 535**
**A1**

(12) # EUROPÄISCHE PATENTANMELDUNG

(21) Anmeldenummer: **88109036.9**

(22) Anmeldetag: **07.06.88**

(51) Int. Cl.4: **C07C 143/18 , C07C 143/12 , B01F 17/08**

(30) Priorität: **15.06.87 DE 3720000**

(43) Veröffentlichungstag der Anmeldung:
**21.12.88 Patentblatt 88/51**

(84) Benannte Vertragsstaaten:
**AT BE CH DE ES FR GB GR IT LI LU NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Behler, Ansgar, Dr.**
**Wittekindstrasse 44**
**D-4250 Bottrop(DE)**
Erfinder: **Piorr, Robert, Dr.**
**Kieselei 12**
**D-4030 Ratingen-Hösel(DE)**
Erfinder: **Schäfer, Michael**
**Rathelbeckerweg 44**
**D-4006 Erkrath(DE)**

(54) **Fettsäurepolyoxyalkylester-sulfonate, Verfahren zu ihrer Herstellung und ihre Verwendung als Tenside.**

(57) Fettsäurepolyoxyalkylester-sulfonate, erhältlich durch Umsetzung von $C_{11}$-$C_{22}$-einfach ungesättigten-Monocarbonsäure-polyoxyalkylestern der allgemeinen Formel I

$C_mH_{2m-1}$-CO-(O-$C_nH_{2n}$)$_x$-OR (I)

in der
R ein Alkylrest mit 1-22 Kohlenstoffatomen oder ein Alkenylrest mit 3-22 Kohlenstoffatomen,
m die Zahlen 10, 15, 17, 19 oder 21,
n die Zahlen 2 und/oder 3 und
x eine Zahl von 1 bis 20 ist,
mit Schwefeltrioxid und anschließender Umsetzung des erhaltenen sulfonierten Fettsäurepolyoxyalkylesters mit wäßrigen Alkalien weisen eine hohe Oberflächenaktivität und gute anwendungstechnische Tensideigenschaften auf.

EP 0 295 535 A1

Xerox Copy Centre

## Fettsäurepolyoxyalkylester-sulfonate, Verfahren zu ihrer Herstellung und ihre Verwendung als Tenside

Die Erfindung betrifft Fettsäurepolyoxyalkylester-sulfonate, erhältlich durch Umsetzung von $C_{11}$-$C_{22}$-einfach ungesättigten-Monocarbonsäure-polyoxyalkylestern der allgemeinen Formel I

$C_mH_{2m-1}$-CO(O-$C_nH_{2n})_x$-OR (I)

in der

R ein Alkylrest mit 1-22 Kohlenstoffatomen oder ein Alkenylrest mit 3-22 Kohlenstoffatomen,
m die Zahlen 10, 15, 17, 19 oder 21,
n die Zahlen 2 und/oder 3 und
x eine Zahl von 1 bis 20 ist,

mit Schwefeltrioxid und anschließender Umsetzung des erhaltenen sulfonierten Fettsäurepolyoxyalkylesters mit wäßrigen Alkalien.

Die technisch wichtigsten anionischen Tenside sind diejenigen, die eine Sulfongruppe als wasserlöslichmachende Gruppe enthalten; bei diesen Tensiden werden Sulfattenside und Sulfonattenside unterschieden.

Sulfattenside sind Halbestersalze der Schwefelsäure. Die wichtigsten Vertreter dieser Untergruppe sind die Alkylethersulfate, die wegen ihrer Glykolethergruppen sehr gut wasserlöslich sind und daher in besonderem Maße zur Herstellung flüssiger Wasch- und Reinigungsmittel geeignet sind. Ein Nachteil dieser Verbindungen ist ihre geringe Hydrolysestabilität, so daß die nicht für saure Reinigungsmittel eingesetzt werden können.

Sulfonattenside sind Salze von Alkylsulfonsäuren, die auch im sauren Medium hydrolysestabil sind.

Sulfonattenside, die zur Verbesserung ihrer Wasserlöslichkeit Glykolethergruppen enthalten, sind die aus der US-PS 1 985 747 bekannten Alkylglykolethersulfonate, die aus der DE-AS 1 075 779 und DE-AS 1 081 172 bekannten Alkylglyceryl-ethersulfonate sowie die aus der DE-OS 33 31 513 bekannten Fettalkyl-(polyoxyalkyl)-niedrigalkyl-ethersulfonate.

Es wurde nun gefunden, daß Fettsäurepolyoxyalkylester-sulfonate, erhältlich durch Sulfonierung von Monocarbonsäure-polyoxyalkylestern der obigen allgemeinen Formel I, sich in einfacher Weise aus leicht zugänglichen Ausgangsmaterialien herstellen lassen; dabei ist überraschend, daß bei der Sulfonierung und Hydrolyse die Esterfunktionen weitgehend erhalten bleiben.

Bevorzugt sind Fettsäurepolyoxyalkylester-sulfonate der Erfindung, bei denen der Fettsäurerest der Formel

$C_mH_{2m-1}$-CO-(O-$C_nH_{2n})_x$-OR

ein von der Ölsäure (m = 17) abgeleiteter Rest ist. Weiterhin kann der vorgenannte Fettsäurerest alternativ von Erucasäure (m = 21) oder von Undecylen(10)-säure (m = 10) abgeleitet sein.

Zweckmäßigerweise verwendet man für die Herstellung der Ausgangsverbindungen der Formel (II) nicht reine Ölsäure bzw. Erucasäure, sondern ölsäure- oder erucasäurereiche Fettsäuregemische, wie sie aus Fetten tierischen, seetierischen und/oder pflanzlichen Ursprungs gewonnen werden können.

Hierfür geeignete typische ölsäurereiche bzw. alternativ einzusetzende erucasäurereiche Fettsäuregemische weisen Zusammensetzungen gemäß der folgenden Tabelle 1 auf.

Undecylen(10)-säure wird großtechnisch in relativ reiner Form aus Ricinolsäure erhalten.

Gemäß einer weiteren, bevorzugten Ausführungsform der Erfindung ist die Gruppe R eine aliphatische Alkylgruppe mit 1-22 Kohlenstoffatomen; besonders bevorzugt sind Gruppen R, die von dem Rest eines gesättigten oder einfach ungesättigten Fettalkohols mit 12-22 Kohlenstoffatomen gebildet ist. Wenn die Gruppe R ein einfach ungesättigter Alkohol, insbesondere Fettalkohol mit 12-22 Kohlenstoffatomen ist, weisen die Ausgangsprodukte der allgemeinen Formel I je eine olefinische Doppelbindung im Säure- und im Alkoholteil auf; in diesen Fällen ist die Einführung von 2 Sulfonatgruppen pro Monocarbonsäure-polyoxyalkylester-Molekül möglich.

Bevorzugt werden zur Herstellung der Ausgangsverbindungen der Formel I solche Fettalkohole eingesetzt, die durch Hydrierung

Tabelle 1

Zusammensetzung üblicher technischer Fettsäuren (in Gew.-%)

| Fettsäure | Ursprung: Rindertalg | Sonnenblume I | Sonnenblume II[1] | Raps |
|---|---|---|---|---|
| $C_{12}$ | 1,0 | - | - | - |
| $C_{14}$ | 3,0 | - | - | 0,5 |
| $C_{15}$ | 0,5 | - | - | - |
| $C_{16}$ | 5,0 | 6,0 | 3,5 | 2 |
| $C_{16}$ : 1 | 6,0 | - | - | - |
| $C_{17}$ | 1,0 | - | - | - |
| $C_{18}$ | 2,0 | 4,0 | 3,5 | 1 |
| $C_{18}$ : 1 | 70,0 | 28 | 85 | 15 |
| $C_{18}$ : 2 | 10,0 | 61 | 7 | 15 |
| $C_{18}$ : 3 | 0,5 | - | - | 7 |
| $C_{20}$ | - | 0,5 | 1 | 0,5 |
| $C_{20}$ : 1 | 1,0 | - | - | 7 |
| $C_{22}$ | - | - | - | 50 |

1) Sonnenblumenöl gemäß US-PS 4 627 192

von aus Fetten tierischen, seetierischen und/oder pflanzlichen Ursprungs gewonnenen technischen Fettsäuregemischen bzw. Alkylestern derselben erhältlich sind.

Die vorgenannten Alkohole mit 1-22 Kohlenstoffatomen werden vor dem Umsetzung mit den Monocarbonsäuren in üblicher Weise mit Ethylenoxid und/oder Propylenoxid polyalkoxyliert. Dabei kann jedes Alkoholmolekül mit 1-20, vorzugsweise 2-12, Polyoxyalkylgruppen umgesetzt werden. Bei der Umsetzung mit Ethylenoxid und Propylenoxid können die Produkte eine block-oder random-Verteilung der Propylenoxidgruppen aufweisen.

Die Erfindung betrifft weiterhin ein Verfahren zur Herstellung von Fettsäurepolyoxyalkylester-sulfonaten durch Umsetzung von einfach ungesättigten $C_1$--$C_{22}$-Monocarbonsäure-polyoxyalkylestern der obigen allgemeinen Formel I, in der R, m, n und x wie oben definiert sind, mit Schwefeltrioxid und anschließender Umsetzung des erhaltenen sulfonierten Fettsäurepolyoxyalkylesters mit wäßrigen Alkalien. Schließlich betrifft die Erfindung die Verwendung der oben beschriebenen Fettsäurepolyoxyalkylester-sulfonate als Tenside.

Die Struktur der Fettsäurepolyoxyalkylester-sulfonate der Erfindung ist noch nicht abschließend geklärt. In Analogie zu sulfonierten Olefinen sind jedoch die folgenden Alken- und Hydroxyalkanstrukturen I und II bzw. Ia und IIa anzunehmen:

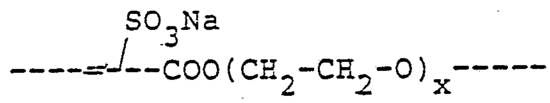

I

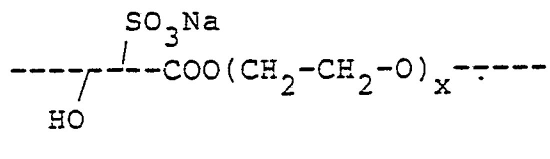

II

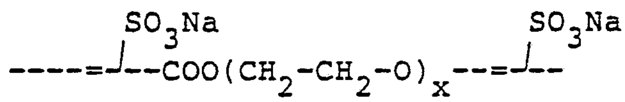

Ia

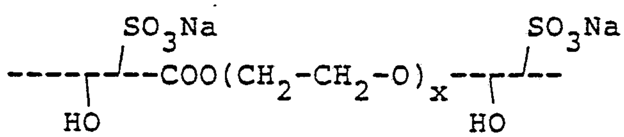

IIa

Die Sulfonierung der $C_{11}$-$C_{22}$-einfach ungesättigten-Monocarbonsäure-polyoxyalkylester der allgemeinen Formel I erfolgt vorzugsweise mit gasförmigem Schwefeltrioxid bei Temperaturen von 20 bis 100 °C. Diese Umsetzung läßt sich in üblichen, für die Sulfonierung von Fettalkoholen, Fettsäureestern, Alkylbenzol oder Olefinen geeigneten Reaktoren, insbesondere vom Typ der Fallfilmreaktoren, kontinuierlich durchführen. Dabei wird das Schwefeltrioxid mit Luft oder Stickstoff verdünnt, vorzugsweise in Form eines Gasgemisches mit ca. 1-10 Vol.-% Schwefeltrioxid, mit den Monocarbonsäure-polyoxyalkylestern der allgemeinen Formel I, besonders bevorzugt bei Temperaturen von 5 bis 40 °C, in Berührung gebracht.

Das rohe Sulfonierungsprodukt wird anschließend in eine wäßrige Lösung eines Alkalihydroxids eingeleitet, insbesondere Natriumhydroxid oder anderer Basen, z.B. Aminen, wobei dieses in einer Menge von 1-1,2 Mol Base pro Mol angelagerten Schwefeltrioxids vorliegen sollte. Der leichte Überschuß an Base dient der Neutralisation des im Sulfonierungsprodukt gelösten gasförmigen Schwefeltrioxids. Bevorzugt wird als Alkalihydroxid Natriumhydroxid eingesetzt; auch Kaliumhydroxid kann verwendet werden. Die Konzentration der Alkalihydroxidlösung wird so gewählt, daß das Endprodukt eine niedrig viskose Lösung bildet.

Das Reaktionsprodukt enthält neben den gewünschten Sulfonaten auch Sultone. Die Bildung von Sultonen ist bei der Sulfonierung olefinischer Doppelbindungen eine an sich bekannte Nebenreaktion, die auch bei dem Verfahren der Erfindung auftritt. Zur Überführung der im Reaktionsprodukt unerwünschten Sultone in Hydroxysulfonate bzw. ungesättigte Sulfonate ist es erforderlich, die wäßrige Lösung einer Hydrolysestufe zu unterwerfen.

Die Hydrolyse wird durch Erwärmen der Lösung unter Aufrechterhaltung eines pH-Wertes von 7 durch kontrollierte Zugabe von Alkalihydroxid bis zur vollständigen Zerstörung der Sultone durchgeführt. Die dafür erforderliche Zeit ist von den Hydrolysebedingungen abhängig. Sie läßt sich z.B. bei Siedetemperatur unter Normaldruck in 4 bis 6 Stunden, bei höheren Temperaturen unter Druck jedoch in erheblich kürzerer Zeit bewerkstelligen, wobei sich der Abschluß der Hydrolysereaktion dadurch bemerkbar macht, daß der pH-Wert des Reaktionsgemisches ohne weitere Zugabe von Alkalihydroxid konstant bleibt.

Die Fettsäurepolyoxyalkylester-sulfonate fallen nach dem Verfahren der Erfindung in Form von dunkel- bis hellgelben wäßrigen, alkalischen Lösungen an. Sie können, falls erwünscht, mit Wasserstoffperoxidlösung oder Chlorlauge in bekannter Weise gebleicht werden.

Zur Stabilisierung gegen Bakterienbefall empfiehlt sich die Konservierung mit aus dem Stand der

Technik bekannten Konservierungsmitteln, z.B. p-Hydroxybenzoat, Sorbinsäure und dergleichen.

Die Ausgangsprodukte der allgemeinen Formel I sind nach literaturbekannten Verfahren zugänglich. Ihre Herstellung geht aus von aliphatischen, gesättigten Alkoholen mit 1-22 Kohlenstoffatomen auf Basis nativer oder synthetischer Alkohole oder einfach ungesättigten Alkoholen mit 3-22 Kohlenstoffatomen, z.B. von Methanol, Ethanol, Propanol, Butanol, Hexanol, Octanol, Decanol; weiterhin von Oleylalkohol oder technischen, überwiegend aus Oleylalkohol, Palmitoleylalkohol und Linoleylalkohol bestehenden Alkoholschnitten. Dabei sind geringe Anteile an gesättigten Alkoholen, z.B. an Cetyl- und Stearylalkohol, unschädlich, insbesondere wenn die aus den Alkoholen durch Oxalkylierung hergestellten Produkte wasserlöslich sind. Geeignete ungesättigte Alkohole sind weiterhin durch Hydrierung von Ölsäure oder von technischen Oleinsäuren herstellbar und im Handel erhältlich. Bevorzugt werden technische Cetyl-Oleyl- und Oleyl-Linoleyl-Alkoholschnittte mit Jodzahlen im Bereich von 70 bis 130.

Die Oxalkylierung der Alkohole mit Ethylenoxid und/oder Propylenoxid ist ein seit langem großtechnisch durchgeführtes Verfahren. Dabei werden Gemische homologer Oxalkylate erhalten, deren mittlerer Oxalkylierungsgrad der Menge des angelagerten Alkylenoxids entspricht.

Die auf diese Weise erhaltenen polyoxalkylierten Alkohole werden anschließend mit den $C_{11}$-$C_{22}$-einfach ungesättigen-Monocarbonsäuren in üblicher Weise umgesetzt, z.B. in Gegenwart von Veresterungskatalysatoren wie Zinnschliff.

Die Fettsäurepolyoxyalkylester-sulfonate der Erfindung weisen eine hohe Oberflächenaktivität und gute anwendungstechnische Tensideigenschaften auf. Besonders günstig ist ihr Benetzungsvermögen gegenüber Textilien. Die gute Wasserlöslichkeit und das befriedigende Emulgiervermögen lassen die Produkte sowohl als technische Netzmittel als auch für die Anwendung in Wasch-und Reinigungsmitteln geeignet erscheinen. Besonders hervorzuheben ist ihre auch ohne Bleichung bereits recht helle Färbung sowie ihre Hydrolysestabilität in alkalischen Medien.

Die Erfindung wird im folgenden anhand von bevorzugten Ausführungsbeispielen näher erläutert.

Beispiel.

Ölsäure-decaglykolethylester-natriumsulfonat.

In einem Labor-Standreaktor wurden 363,3g (0,5 Mol) Ölsäure-decaglykolethylester auf 30 °C erwärmt; innerhalb von 50 min wurden 40 g Schwefeltrioxid (0,5 Mol; erzeugt durch Verdampfen von 65 %-igem Oleum), verdünnt mit Stickstoff (5 Vol.-% $SO_3$ im Stickstoffstrom), eingeleitet. Nach einer 30-minütigen Nachreaktion wurde das saure Sulfonierungsprodukt gleichzeitig mit einer wäßrigen 25 %-igen Natriumhydroxidlösung in vorgelegtes Wasser gegeben. Anschließend wurde auf 90 °C erhitzt und so lange NaOH zugegeben, bis sich ein konstanter pH-Wert von 7 einstellte. Insgesamt wurde 0,51 Mol Natriumhydroxid verbraucht. Das erhaltene Reaktionsprodukt war in Wasser klar löslich (30 %-ig).

Die chemisch-physikalischen Kenndaten der so erhaltenen Verbindung sind mit denjenigen weiterer, analog zu dem Verfahren des Ausfuhrungsbeispiels hergestellter Verbindungen in Tabelle 2 zusammengefaßt.

Tabelle 3 enthält die Verseifungszahlen (VZ) einiger der hergestellten Verbindungen, und zwar VZ ist, VZ soll sowie den Quotienten VZ ist/VZ soll.

Aus Tabelle 3 zeigt sich deutlich, daß die Esterbindung bei der Sulfonierung und der anschließenden Neutralisation und Hydrolyse überweigend erhalten geblieben ist.

Tabelle 2

$C_{17}H_{33}-CO-(CH_2CH_2O)_x-OR \cdot SO_3Na$

| Verbindung Nr. | x | R | WAS(%)[1] | $Na_2SO_4$ (Gew.-%) | Farbzahl[2] | Aktivsubstanz[4] (O) |
|---|---|---|---|---|---|---|
| 1 | 10 | $C_2H_5$ | 23,7 | 0,6 | 29 | 35,1 |
| 2 | 2,9 | $C_{10}H_{21}$ | 19,8 | 0,7 | 45 | 25,3 |
| 3 | 7 | $C_{12}/C_{14}$ | 28,9 | 0,4 | | 29,6 |
| 4 | 3 | $C_2H_5$ | 23,0 | 1,7 | | 32,0 |
| 5 | 4 | $C_8H_{17}$ | 18,8 | 0,6 | | 25,0 |
| 6 | 5 | $C_{16}/C_{18}$,[3] | 25,8 | 1,4 | | |

1) Waschaktivsubstanz (DGF-Methode H-III-10)
2) Klettfarbzahl (NaOCl-Bleiche; 1 cm-Küvette)
3) Gemisch aus Myristyl- und Oleylalkohol
4) Trockenrückstand

Tabelle 3

| Verbindung Nr. | VZ ist | VZ soll | VZ ist / VZ soll |
|---|---|---|---|
| 1 | 66,49 | 67,6 | 0,98 |
| 3 | 47,25 | 60,1 | 0,71 |
| 4 | 93,89 | 103,5 | 0,91 |

**Ansprüche**

1. Fettsäurepolyoxyalkylester-sulfonate, erhältlich durch Umsetzung von $C_{11}$-$C_{22}$-einfach ungesättigten-Monocarbonsäurepolyalkylestern der allgemeinen Formel I

$C_mH_{2m-1}$-CO-(O-$C_nH_{2n}$)$_x$-OR (I)

in der

R ein Alkylrest mit 1-22 Kohlenstoffatomen oder ein Alkenylrest mit 3-22 Kohlenstoffatomen,
m die Zahlen 10, 15, 17, 19 oder 21,
n die Zahlen 2 und/oder 3 und
x eine Zahl von 1 bis 20 ist,
mit Schwefeltrioxid und anschließender Umsetzung des erhaltenen sulfonierten Fettsäurepolyoxyalkylesters mit wäßrigen Alkalien.

2. Fettsäurepolyoxyalkylester-sulfonate nach Anspruch 1, dadurch gekennzeichnet, daß der Fettsäurerest der Formel

$C_mH_{2m-1}$-CO-

ein von Ölsäure (m = 17) abgeleiteter Rest ist.

3. Fettsäurepolyoxyalkylester-sulfonate nach Anspruch 1, dadurch gekennzeichnet, daß der Fettsäurerest der Formel

$C_mH_{2m-1}$-CO-

ein von Erucasäure (m = 21) gebildeter Rest ist.

4. Fettsäurepolyoxyalkylester-sulfonate nach einem der Ansprüche 1 bis 3, dadurch gekennzeichnet, daß die $C_{11}$-$C_{22}$-einfach ungesättigten-Monocarbonsäure-polyoxyalkylester aus von Fetten tierischen, seetierischen und/oder pflanzlichen Ursprungs gewonnenen, ölsäure- oder erucasäurereichen Fettsäuregemischen erhältlich sind.

5. Fettsäurepolyoxyalkylester-sulfonate nach einem der Ansprüche 1 bis 4, dadurch gekennzeichnet, daß x eine Zahl von 2 bis 12 ist.

6. Fettsäurepolyoxyalkylester-sulfonate nach einem der Ansprüche 1 bis 5, dadurch gekennzeichnet, daß die Gruppe R eine aliphatische Alkylgruppe mit 1-22 Kohlenstoffatomen ist.

7. Fettsäurepolyoxyalkylester-sulfonate nach einem der Ansprüche 1 bis 5, dadurch gekennzeichnet, daß die Gruppe R von dem Rest eines gesättigten oder einfach ungesättigten Fettalkohols mit 12-22 Kohlenstoffatomen gebildet ist.

8. Fettsäurepolyoxyalkylester-sulfonate nach einem der Ansprüche 1 bis 7, dadurch gekennzeichnet, daß die die Gruppe R aufweisenden Fettalkohole durch Hydrierung von aus von Fetten tierischen, seetierischen und/oder pflanzlichen Ursprungs gewonnenen technischen Fettsäuregemischen bzw. Alkylestern derselben erhältlich sind.

9. Verfahren zur Herstellung von Fettsäurepolyoxyalkylester-sulfonaten, dadurch gekennzeichnet, daß man $C_{11}$-$C_{22}$-einfach ungesättigte-Monocarbonsäure-polyoxyalkylester der allgemeinen Formel I

$C_mH_{2m-1}$-CO-(O-$C_nH_{2n}$)$_x$-OR (I)

in der
R ein Alkylrest mit 1-22 Kohlenstoffatomen oder ein Alkenylrest mit 3-22 Kohlenstoffatomen,
m die Zahlen 10, 15, 17, 19 oder 21,
n die Zahlen 2 und/oder 3 und
x eine Zahl von 1-20 bedeutet,
mit Schwefeltrioxid und anschließend den so erhaltenen sulfonierten Fettsäurepolyoxyalkylester mit wäßrigen Alkalien umsetzt.

10. Verfahren nach Anspruch 9, dadurch gekennzeichnet, daß man $C_{11}$-$C_{22}$-einfach ungesättigte-Monocarbonsäure-polyoxyalkylester verwendet, deren Fettsäurerest der Formel

$C_mH_{2m-1}$-CO-

ein von Ölsäure (m = 17) abgeleiteter Rest ist.

11. Verfahren nach Anspruch 9, dadurch gekennzeichnet, daß man $C_{11}$-$C_{22}$-einfach ungesättigte-Monocarbonsäure-polyoxyalkylester verwendet, deren Fettsäurerest der Formel

$C_mH_{2m-1}$-CO-

ein von Erucasäure (m = 21) abgeleiteter Rest ist.

12. Verfahren nach einem der Ansprüche 9 bis 11, dadurch gekennzeichnet, daß man $C_{11}$-$C_{22}$-einfach ungesättigte-Monocarbonsäure-polyoxyalkylester verwendet, die aus Fetten tierischen, seetierischen, und/oder pflanzlichen Ursprungs gewonnenen, ölsäure- oder erucasäurereichen Fettsäuregemischen erhältlich sind.

13. Verfahren nach einem der Ansprüche 9 bis 12, dadurch gekennzeichnet, daß man $C_{11}$-$C_{22}$-einfach ungesättigte-Monocarbonsäure-polyoxyalkylester der allgemeinen Formel I verwendet, in der x eine Zahl von 2 bis 12 ist.

14. Verfahren nach einem der Ansprüche 9 bis 13, dadurch gekennzeichnet, daß man $C_{11}$-$C_{22}$-einfach ungesättigte-Monocarbonsäure-polyoxyalkylester der allgemeinen Formel I verwendet, in der die Gruppe R eine aliphatische Alkylgruppe mit 1-22 Kohlenstoffatomen ist.

15. Verfahren nach einem der Ansprüche 9 bis 14, dadurch gekennzeichnet, daß man $C_{11}$-$C_{22}$-einfach ungesättigte-Monocarbonsäure-polyoxyalkylester der allgemeinen Formel I verwendet, in der die Gruppe R von dem Rest eines gesättigten oder einfach ungesättigten Fettalkohols mit 12-22 Kohlenstoffatomen gebildet ist.

16. Verfahren nach Anspruch 9, dadurch gekennzeichnet, daß man $C_{11}$-$C_{22}$-einfach ungesättigte-Monocarbonsäure-polyoxyalkyl ester der allgemeinen Formel I verwendet, in denen die Gruppe R von Fettalkoholen abgeleitet ist, die durch Hydrierung von aus Fetten tierischen, seetierischen und/oder pflanzlichen Ursprungs gewonnenen technischen Fettsäuregemischen bzw. Alkylestern derselben erhältlich sind.

17. Verwendung von Fettsäurepolyalkylester-sulfonaten erhältlich durch Umsetzung von $C_{11}$-$C_{22}$-einfach ungesättigten-Monocarbonsäuren-polyalkylestern der allgemeinen Formel I

$C_mH_{2m-1}$-CO-(O-$C_nH_{2n}$)$_x$-OR (I)

in der

R ein Alkylrest mit 1-22 Kohlenstoffatomen oder ein Alkenylrest mit 3-22 Kohlenstoffatomen,

m die Zahlen 10, 15, 17, 19 oder 21,

n die Zahlen 2 und/oder 3 und

x eine Zahl von 1 bis 20 ist,

mit Schwefeltrioxid und anschließender Umsetzung des erhaltenen sulfonierten Fettsäurepolyoxyalkylesters mit wäßrigen Alkalien, als Tenside.

18. Verwendung nach Anspruch 17, dadurch gekennzeichnet, daß der Fettsäurerest der Formel

$C_mH_{2m-1}$-CO-

ein von Ölsäure (m = 17) abgeleiteter Rest ist.

19. Verwendung nach Anspruch 17, dadurch gekennzeichnet, daß der Fettsäurerest der Formel

$C_mH_{2m-1}$-CO-

ein von Erucasäure (m = 21) gebildeter Rest ist.

20. Verwendung nach einem der Ansprüche 17 bis 19, dadurch gekennzeichnet, daß die $C_{11}$-$C_{22}$-einfach ungesättigten-Monocarbonsäure-polyoxyalkylester aus von Fetten tierischen, seetierischen und/oder pflanzlichen Ursprungs gewonnenen, ölsäure- oder erucasäurereichen Fettsäuregemischen erhältlich sind.

21. Verwendung nach einem der Ansprüche 17 bis 20, dadurch gekennzeichnet, daß x eine Zahl von 2 bis 12 ist.

22. Verwendung nach einem der Ansprüche 17 bis 21, dadurch gekennzeichnet, daß die Gruppe R eine aliphatische Alkylgruppe mit 1-22 Kohlenstoffatomen ist.

23. Verwendung nach einem der Ansprüche 17 bis 22, dadurch gekennzeichnet, daß die Gruppe R von dem Rest eines gesättigten oder einfach ungesättigten Fettalkohols mit 12-22 Kohlenstoffatomen gebildet ist.

24. Verwendung nach einem der Ansprüche 17 bis 23, dadurch gekennzeichnet, daß die die Gruppe R aufweisenden Fettalkohole durch Hydrierung von aus von Fetten tierischen tierischen, seetierischen und/oder pflanzlichen Ursprungs gewonnenen technischen Fettsäuregemischen bzw. Alkylestern derselben erhältlich sind.

Europäisches Patentamt

# EUROPÄISCHER RECHERCHENBERICHT

Nummer der Anmeldung

EP 88 10 9036

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| | Keine Entgegenhaltungen.<br>----- | | C 07 C 143/18<br>C 07 C 143/12<br>B 01 F 17/08 |
| | | | RECHERCHIERTE SACHGEBIETE (Int. Cl.4) |
| | | | C 07 C 143/00<br>C 08 G 65/00 |

Der vorliegende Recherchenbericht wurde für alle Patentansprüche erstellt

| Recherchenort | Abschlußdatum der Recherche | Prüfer |
|---|---|---|
| DEN HAAG | 09-09-1988 | VAN GEYT J.J.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)